Europäisches Patentamt

**(19)** European Patent Office

Office européen des brevets

**(11)** Publication number: **0 221 609**
**A1**

**(12)** # EUROPEAN PATENT APPLICATION

**(21)** Application number: **86201884.3**

**(22)** Date of filing: **28.10.86**

**(51)** Int. Cl.⁴: **C 12 N 15/00**
**A 61 K 39/215, C 07 K 13/00**
**C 12 N 1/20, C 12 P 21/02**

**(30)** Priority: **31.10.85 NL 8502975**

**(43)** Date of publication of application:
**13.05.87 Bulletin 87/20**

**(84)** Designated Contracting States:
**AT BE CH DE ES FR GB GR IT LI LU NL SE**

**(71)** Applicant: **DUPHAR INTERNATIONAL RESEARCH B.V**
**C.J. van Houtenlaan 36**
**NL-1381 CP Weesp(NL)**

**(72)** Inventor: **Niesters, Hubert G.M.**
**c/o OCTROOIBUREAU ZOAN B.V. P.O. Box 140**
**NL-1380 AC Weesp(NL)**

**(72)** Inventor: **van der Zeijst, Bernardus A.M.**
**c/o OCTROOIBUREAU ZOAN B.V. P.O. Box 140**
**NL-1380 AC Weesp(NL)**

**(74)** Representative: **Muis, Maarten et al,**
**OCTROOIBUREAU ZOAN B.V. P.O. Box 140**
**NL-1380 AC Weesp(NL)**

**(54)** New antigenically active proteins and peptides, and infectious bronchitis virus (IBV) vaccines.

**(57)** The invention relates to new antigenic proteins which can stimulate the immunity of poultry against infectious bronchitis (IB) viruses, and to fragments or derivatives thereof. The invention also relates to vaccines which comprise such an antigenic protein or peptide.

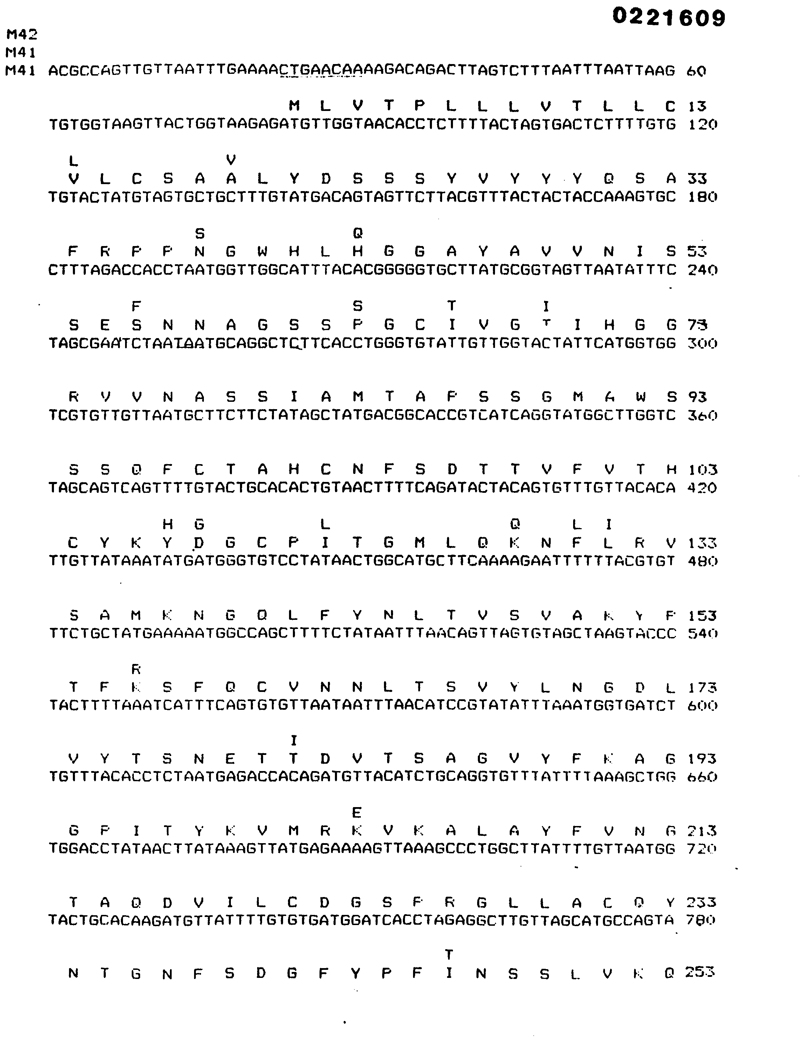

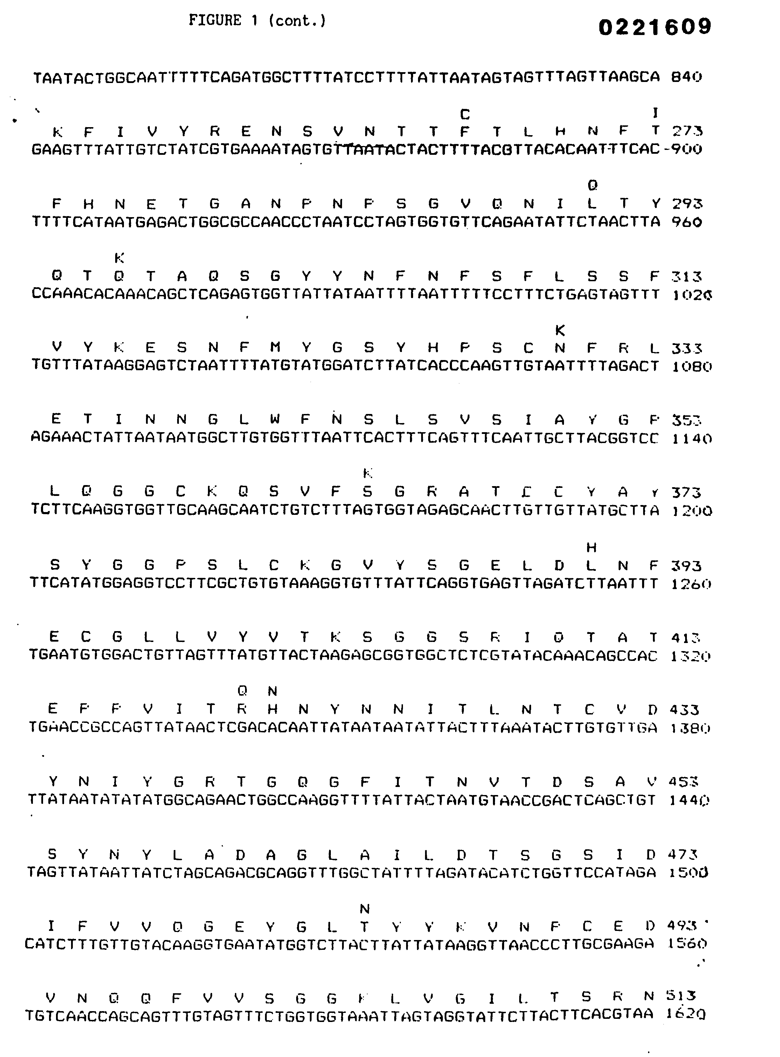

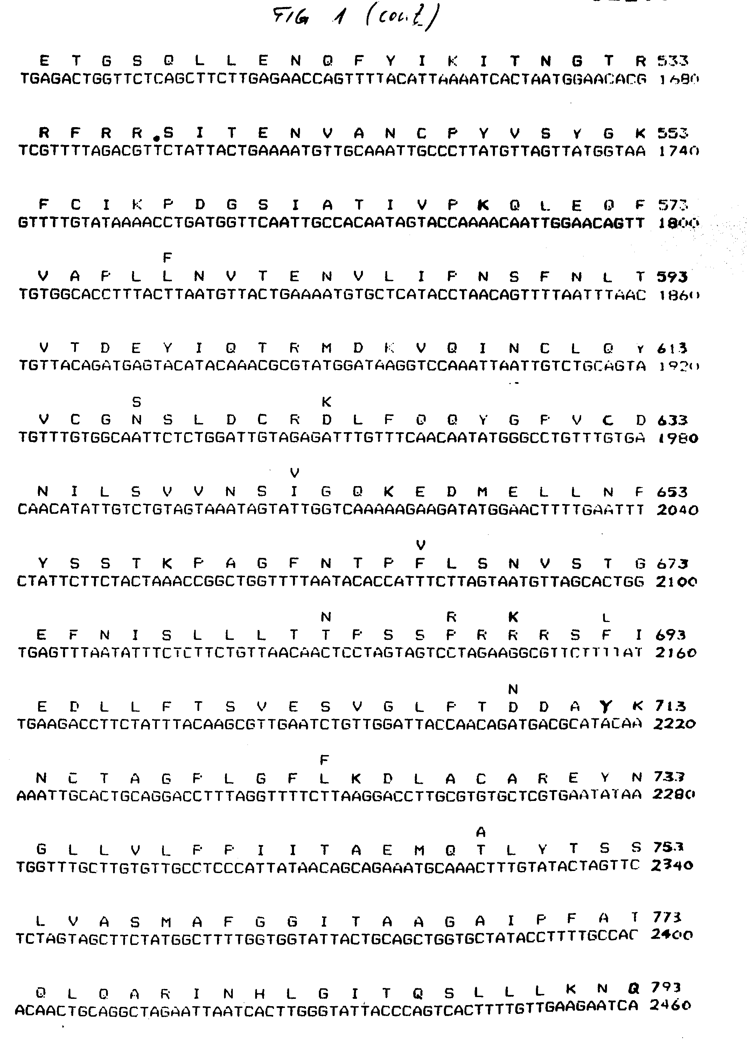

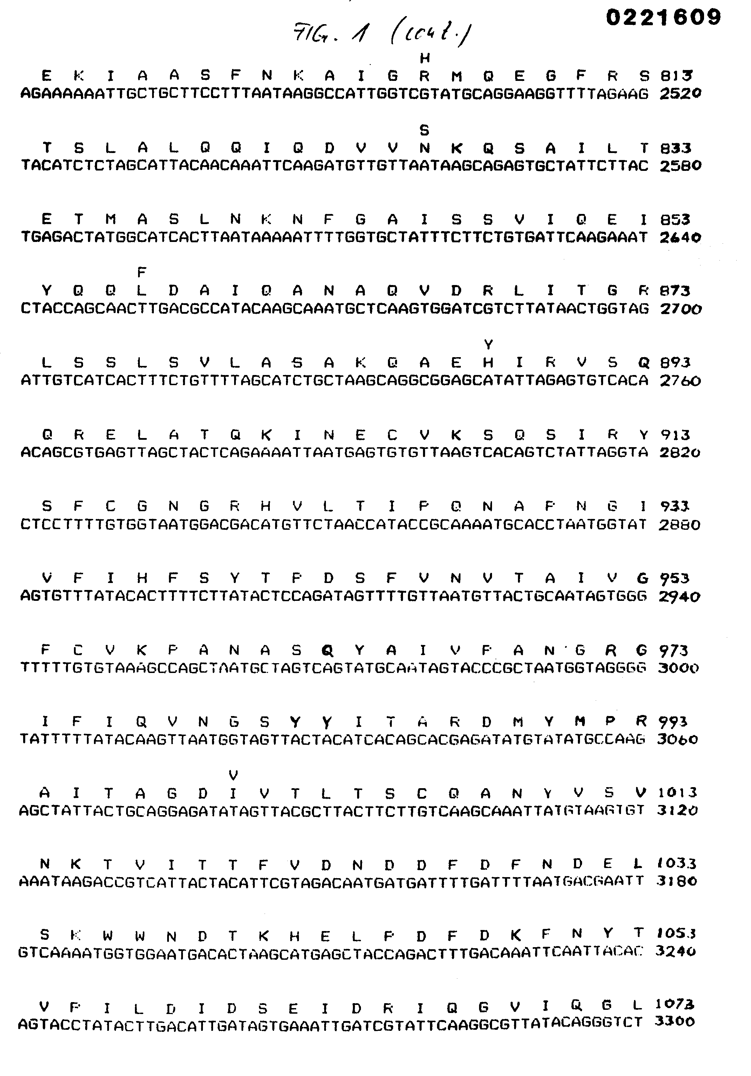

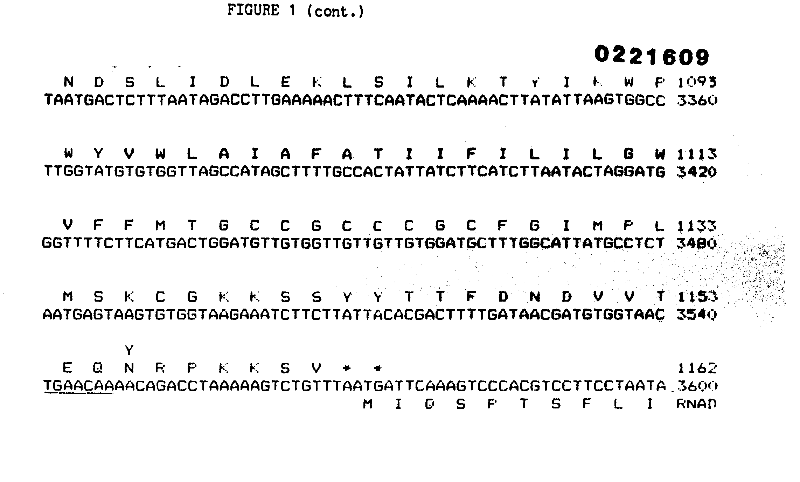

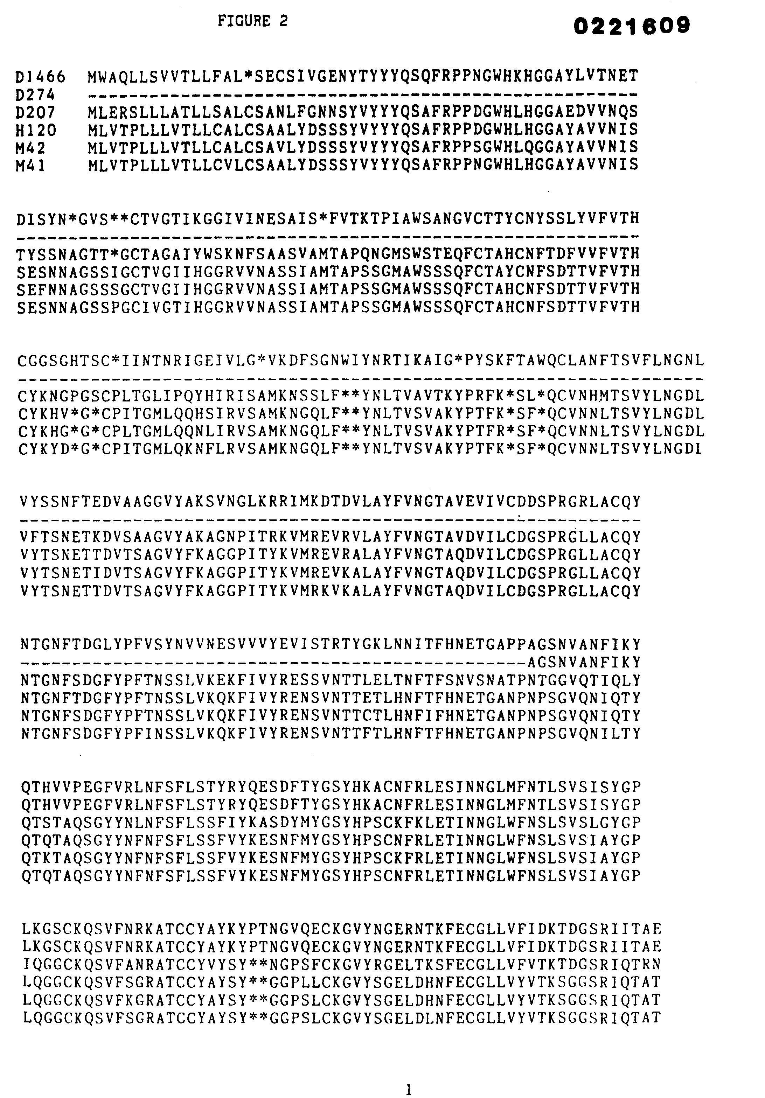

A nucleotide sequence and amine acid sequence for a gene coding for protein of an IBV strain are indicated in figure 1 and 2 respectively.

EP 0 221 609 A1

New antigenically active proteins and peptides, and infectious bronchitis virus (IBV) vaccines.

The invention relates to new antigenic proteins which can stimulate the immunity of poultry against infectious bronchitis viruses (IBV), and to fragments or derivatives thereof. The invention furthermore relates to vaccines which comprise such an antigenic protein or peptide.

Infectious bronchitis still causes considerable mortality especially in young poultry, inspite of the availability of various types of vaccines. The development of new and better vaccines is hence urgently necessary.

IBV belongs to the corona viruses. Corona viruses are positive single-stranded RNA viruses with a lipid envelope. The genome RNA has a length of approximately 20,000 bases. The virus particle (virion) comprises three important proteins, namely the peplomeric protein (S), the nucleocapside protein (N), and the matrix protein (M).

The characteristic projections at the surface of the virion are formed by the peplomeric protein (S). Each peplomer consists of the two glycopolypeptides $S_1$ and $S_2$. The $S_2$ protein is fixed in the membrane, and the $S_1$ protein is coupled _via_ the $S_2$ protein.

Recently the nucleotide sequence was determined of the laboratory IBV-strain Beaudette (or strain M42). However, it was established that neutralizing antibodies against this Beaudette strain are not capable of protecting chickens against either other viruses of the serotype Massachusetts, for example, the strains H120 and M41, nor against the Netherlands field isolates D1466, D207 and D274.

According to the present invention, the nucleotide sequence and the amino acid sequence were determined of the strains M41, D1466, D207 and D274 which strains are used in practice in vaccines to protect chickens against IBV infections. By comparing the nucleotide sequence and amino acid sequence of vaccine strains kM41, D1466, D207 and D274 according to the invention with each other and with the known nucleic acid sequence and the amino acid sequence of strain M42, it is possible now to determine those regions of the protein which are of importance for generating protecting antibodies from the differences between the sequences.

The invention therefore relates to proteins and fragments thereof which comprise at least one of the regions in which the most important differences are located between the amino acid sequences of M42 and M41, D 1466, D207 and/or D274 respectively. It has been found that these differences are found in particular in two regions of the $S_1$ protein.

Theoretically, such fragments can be used as such as an antigen for vaccinating against IBV. However, it is to be preferred to couple these fragments to a suitable carrier.

The peptide fragments or proteins according to the invention which comprise at least one antigenically active peptide fragment can be prepared according to methods known for the preparation of peptides and proteins.

First of all, the peptides can be prepared synthetically by means of known techniques starting from the individual amino acids or smaller peptide fragments.

Alternatively, the peptides can also be obtained biosynthetically using recombinant DNA techniques and expression systems, for example, by

a) transformation of host cells with an expression vector which comprises a DNA, coding for an (the) antigenic determinant(s) (peptides in general);

b) expressing the genome introduced into the expression vector;

c) harvesting the cell culture, and

d) separating the synthesized peptide (protein).

The invention therefore also relates to a method of preparing a DNA molecule which codes for peptides according to the invention. Such a method comprises the following steps:

a) isolation of IBV single-stranded RNA,

b) synthesis of a cDNA strand which is complementary to the RNA strand mentioned in a), and

c) removal of the RNA molecule and synthesis of a second cDNA strand, with the first cDNA strand as template.

The double-stranded DNA obtained in this manner can be incorporated in known manner in an expression vector, as a result of which a recombinant expression vector is formed. This vector can be incorporated into a suitable host cell, for example, by transformation.

The invention will now be described in greater detail with reference to the ensuing specific examples.

EXAMPLE I

a) Preparation of viral RNA.

Virus of the known IBV vaccine strain M41 (originating from the Stichting Gezondheidszorg voor Pluimvee (Foundation Health Service for Poultry) in Doorn, the Netherlands) was cloned twice by end point dilution in 10-day-old chicken embryos.

Approximately 100 $EID_{50}$ of the virus was inocculated in the allantoic cavity of 10-day-old chicken embryos and incubated at 37°C for 40 hours. The eggs were cooled at 4°C for at least 4 hours before the allantoic fluid was harvested. After a first purification, sodium chloride and polyethylene glycol 6000 were added to a final concentration of 2.33 and 10% (wt-vol), respectively. The mixture was stirred overnight at 4°C and centrifugated for 30 minutes (10,000 g). The solid fraction and the floating fat fraction were resuspended in a quantity of TESV-buffer (0.02M Tris-HCl, 1mM EDTA, 0.1M NaCl, pH 7.4) corresponding to 1/50 of the original volume. The concentrated virus suspension was clarified and layered onto 10 ml of 10% (wt--vol) sucrose solution on top of 16 ml of 20-50% (wt-vol) sucrose gradient, both in TESV buffer, and centrifugated (24,000 rpm) in an SW 27.0 rotor for 6 hours).

The virus-containing band was collected and diluted in TESV buffer. The virus was pelleted, at 4°C for 90 minutes (250,000 g). The pellet was incubated at 37°C for 30 minutes with 200 /ul of TESV containing 0.5% of SDS and 0.5 mg/ml of proteinase K. The RNA was finally obtained by extraction with phenol/chloroform and ether, and precipitation with methanol.

### b) Cloning of IBV genome RNA.

The synthesis of cDNA was carried out according to the method of Gubler and Hoffman (Gene, 25 (1983), 263-269) without the addition of T4 DNA ligase. Double-stranded cDNA was tailed with dC residues and cloned into dG tailed PstI-cleaved pUC9 and subsequently transformed into E.coli JM109. White clones were isolated and characterized.

Clones comprising viral sequences were identified by hybridisation with a full-length IBV RNA probe.

### c) DNA sequencing.

Fragments were isolated using NA-45 paper and again cloned in M13mp9 (Schleicher and Schuell). Sequencing was carried out by means of the dideoxy method (Sanger et al, Proc. Natl. Acad. Sci. USA, 74 (1977), 5463-5467) using alpha-$^{32}$P-dATP (Amersham). Both the universal M13 sequencing primer and internal synthetic primers (having a length of 15 nucleotides) were used. The complete sequence was determined on both strains via independently isolated cDNA clones. The data were analysed on a DEC 20/60 computer by using the programs of Staden, Nucleic Acids Research, 10, (1982), 4731-4751).

### d) Nucleotide sequence.

An oligonucleotide primer which is complementary to the 5' end of the gene encoding the membrane protein of the Beaudette strain (M42) was used for the preparation of cDNA against the M41 strain. Forty-eight clones were further characterised. One of them, p39, comprised a viral insert of 4,000 bases which comprises the genetic information of the peplomeric protein. Restriction map analysis and Southern blotting were used to isolate independent DNA clones comprising the p39 sequences.

The nucleotide sequence of the gene which codes for the peplomeric protein of the IBV strain M41, as well as the regions homologous to M42 are shown in Figure 1. The sequence has an open reading frame of 3486 nucleotides with a coding capacity for a precursor polypeptide with a molecular weight of 128.079 (1162 amino acids).

Around the first putative initiation codon used sequences are located which meet the rules of Kozak (Nucl. Acids. Res. 12 (1984), 856-872).

### e) Amino acid sequence.

In the Beaudette strain the signal sequence of 18 amino acids is cleaved between alanine and valine.

The signal sequence of the strain M41 comprises the same region of preferences for particular amino acids at particular positions  However, an alanine is present at position 19. This might indicate that the signal sequence is preferably removed by cleavage between Ala 18 and Ala 19, but that an alternative cleavage site is present between Ala 19 and Leu 20 (Von Heijne, J. Mol. Biol. 173 (1984), 243-251).

The N terminal amino acid of the $S_2$ protein is Ser 538, next to two basic residues. It is possible that the polypeptide is not split only at the previous mentioned positions, but also between Thr 532 and Arg 533, which involves excision of a peptide Arg-Arg-Phe-Arg-Arg. The S precursor hence starts with a signal sequence of 18-19 amino acids, followed by an $S_1$ chain consisting of at most 519 residues, and an $S_2$ peptide consisting of at most 625 residues.

Comparison of the amino acid sequences of the strains M41 and M42 shows a high degree of homology (96.2%).

The protein has 29 potential glycosylation sites, 17 in the $S_1$ unit and 12 in the $S_2$ unit.

## f) Comparison of IBV peplomeric proteins.

A comparison of the amino acid sequences of the peplomeric proteins of the strains M41 and M42 is shown in figure 1. Both proteins have the same length. There is a potential extra glycosylation site in the $S_1$ protein of M41. Antibodies against the $S_1$ protein are able to neutralize the virus. Therefore amino acid substitutions must be responsible for the antigenic drift of the virus strains. There are 25 amino acid changes, 23 of which have occurred via single base changes, and the remaining two by double mutations (Pro 63 and Lys 128).

Two regions with clustered amino acid substitutions are present in the $S_1$ protein (see figure 1), namely residues 56-70 and 116-131, and only one region with clustered mutations (679-692) is present in the $S_2$ protein.

In a similar manner as described in Example I a) to e) the amino acid sequence of the known IBV strain H120 and of the IBV strains D1466, D207 and D274 (obtained from the Foundation Health Service for Poultry in Doorn, the Netherlands) were determined. The amino acid sequences of these strains and of the strains M41 and M42 are indicated in figure 2.


EXAMPLE II
Preparation of peptides


a) By means of organic synthesis reactions:

By means of techniques known per se, the following peptides of strain M41 were synthesised by means of the solid phase method: 56-70, 116-131, 679-692, and 1043-1055 (see figure 1).


b) By cloning and expression in bacterial expression vectors.

cDNA was made by means of standard techniques starting from IBV-RNA as template.

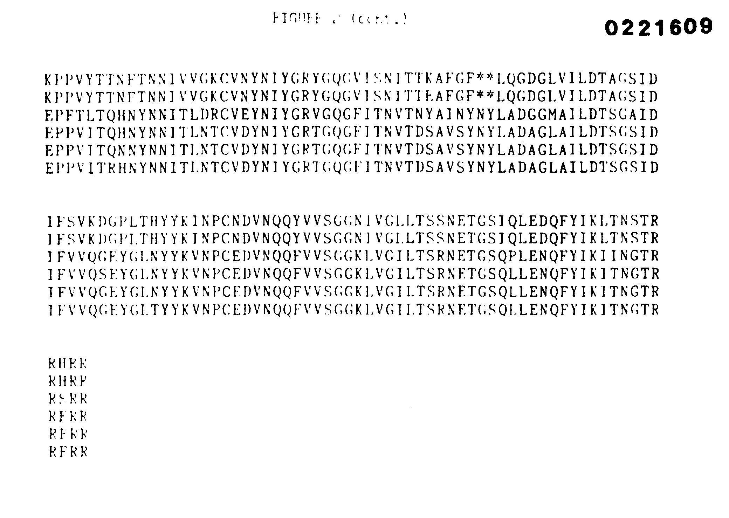

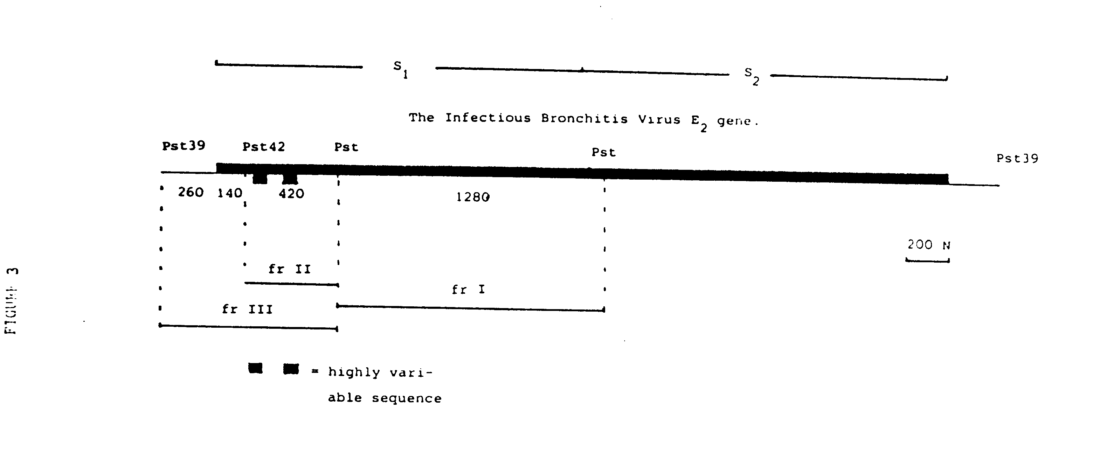

From the cDNA clones 39 and 42, the fragments I, III and II were isolated, likewise according to standard techniques, with restriction-endonuclease Pst I (see figure 3).

These Pst I fragments were cloned in the Pst I site of the pEX-expression vectors which were constructed by Stanley from the EMBL (EMBO Journal, vol. 3, No. 6 (1984), pp. 1429-1434 and Vol. 1, No. 10 (1982), pp. 1217-1224). Cloning and transformation were carried out according to standard techniques and cultivation was carried out at 30°C.

Preselection of clones took place by bringing the transformation mix on ampicilline-containing plates (50 /ug/ml).

8

Selection was carried out by transferring ampicilline-resistant clones to nitrocellulose, cultivating and
examining for IBV specific proteins by means of immuno-
screening. For this purpose a 1/1000 diluted polyclonal rabbit serum against IBV M41 was used. Positive clones isolated, and protein expression was induced by temperature
shift from 30°C to 42°C as described by Stanley (see
above).

In figure 1, each time three lines belong together.

The nucleotide sequence found for M41 is shown in the
bottom line. The centre line indicates the amino acid sequence of M41, and the line on top gives the position where
M42 and M41 are different.

CLAIMS:

1 A gene which for protein of an IBV strain, characterized in that it codes for protein of IBV strains M41, D1466, D207 or D274 having the amino acid sequences indicated in figure 2.

2. A protein, characterized by the amino acid sequence as shown in figure 2, or a part thereof.

3. A gene coding for protein of an IBV strain, characterized by the nucleotide sequence as shown in figure 1, or a part thereof.

4. A peptide, characterized in that it comprises at least one of the regions in which the amino acid sequences of the IBV strains M41 and M42 are different.

5. A peptide as claimed in Claim 4, characterized in that it comprises the region between the amino acids 55 and 70 and/or between the amino acids 116 and 131 and/or between the amino acids 290 and 301.

6. An immunogen, characterized in that it comprises a protein or peptide as claimed in Claim 2, 4 or 5, coupled to a carrier.

7. A vaccine, characterized in that it comprises a protein, peptide or immunogen as claimed in claim 2, 4, 5 or 6.

8. A method of preparing a protein or peptide as claimed in Claim 2, 4 or 5, characterized in that:
a) the protein or peptide is synthesised in a manner known per se from individual amino acids and/or by coupling of smaller peptides; or
b) a host cell is transformed with an expression vector which comprises a DNA coding for a protein or peptide as claimed in Claim 2, 4 or 5, and the genome introduced into the expression vector is expressed.

9. A DNA molecule which comprises a nucleotide sequence which codes for a protein or peptides as claimed in Claim 2, 4 or 5, or which codes for a polypeptide which comprises a protein or peptide as claimed in Claim 2, 4 or 5.

10. A recombinant DNA expression vector which expresses the whole protein or peptide or a part thereof as defined in Claims 2, 4 or 5, comprising an operon with initiator sequences and terminator sequences and a nucleotide sequence being between the initiator sequences and the terminator sequences of the operon.

11. A host cell which comprises a recombinant DNA expression vector and/or a DNA molecule as claimed in Claim 9 or 10.

12. A method of preparing a DNA molecule which codes for a protein or peptide as claimed in claims 2, 4 or 5, characterized in that

a) single-stranded IBV-RNA is isolated,

b) a cDNA strand complementary to said RNA strand is synthesised,

c) the RNA strand is removed and a second strand cDNA is synthesised using the first cDNA strans as template.

FIGURE 1

**0221609**

```
M42
M41
M41 ACGCCAGTTGTTAATTTGAAAACTGAACAAAAGACAGACTTAGTCTTTAATTTAATTAAG 60

                              M  L  V  T  P  L  L  L  V  T  L  L  C 13
    TGTGGTAAGTTACTGGTAAGAGATGTTGGTAACACCTCTTTTACTAGTGACTCTTTTGTG 120

       L                 V
       V  L  C  S  A  A  L  Y  D  S  S  S  Y  V  Y  Y  Y  Q  S  A 33
    TGTACTATGTAGTGCTGCTTTGTATGACAGTAGTTCTTACGTTTACTACTACCAAAGTGC 180

                 S                    Q
       F  R  P  P  N  G  W  H  L  H  G  G  A  Y  A  V  V  N  I  S 53
    CTTTAGACCACCTAATGGTTGGCATTTACACGGGGGTGCTTATGCGGTAGTTAATATTTC 240

          F                             S           T           I
       S  E  S  N  N  A  G  S  S  P  G  C  I  V  G  T  I  H  G  G 73
    TAGCGAATCTAATAATGCAGGCTCTTCACCTGGGTGTATTGTTGGTACTATTCATGGTGG 300

       R  V  V  N  A  S  S  I  A  M  T  A  P  S  S  G  M  A  W  S 93
    TCGTGTTGTTAATGCTTCTTCTATAGCTATGACGGCACCGTCATCAGGTATGGCTTGGTC 360

       S  S  Q  F  C  T  A  H  C  N  F  S  D  T  T  V  F  V  T  H 103
    TAGCAGTCAGTTTTGTACTGCACACTGTAACTTTTCAGATACTACAGTGTTTGTTACACA 420

             H  G             L                    Q     L  I
       C  Y  K  Y  D  G  C  P  I  T  G  M  L  Q  K  N  F  L  R  V 133
    TTGTTATAAATATGATGGGTGTCCTATAACTGGCATGCTTCAAAAGAATTTTTTACGTGT 480

       S  A  M  K  N  G  Q  L  F  Y  N  L  T  V  S  V  A  K  Y  P 153
    TTCTGCTATGAAAAATGGCCAGCTTTTCTATAATTTAACAGTTAGTGTAGCTAAGTACCC 540

             R
       T  F  K  S  F  Q  C  V  N  N  L  T  S  V  Y  L  N  G  D  L 173
    TACTTTTAAATCATTTCAGTGTGTTAATAATTTAACATCCGTATATTTAAATGGTGATCT 600

                      I
       V  Y  T  S  N  E  T  T  D  V  T  S  A  G  V  Y  F  K  A  G 193
    TGTTTACACCTCTAATGAGACCACAGATGTTACATCTGCAGGTGTTTATTTTAAAGCTGG 660

                         E
       G  P  I  T  Y  K  V  M  R  K  V  K  A  L  A  Y  F  V  N  G 213
    TGGACCTATAACTTATAAAGTTATGAGAAAAGTTAAAGCCCTGGCTTATTTTGTTAATGG 720

       T  A  Q  D  V  I  L  C  D  G  S  F  R  G  L  L  A  C  Q  Y 233
    TACTGCACAAGATGTTATTTTGTGTGATGGATCACCTAGAGGCTTGTTAGCATGCCAGTA 780

                               T
       N  T  G  N  F  S  D  G  F  Y  P  F  I  N  S  S  L  V  K  Q 253
```

DUPHAR INTERNATIONAL RESEARCH B.V.

DIR 0370

FIGURE 1 (cont.)

0221609

```
TAATACTGGCAATTTTTCAGATGGCTTTTATCCTTTTATTAATAGTAGTTTAGTTAAGCA 840

                                              C                 I
    K  F  I  V  Y  R  E  N  S  V  N  T  T  F  T  L  H  N  F  T 273
GAAGTTTATTGTCTATCGTGAAAATAGTGTTAATACTACTTTTACGTTACACAATTTCAC -900


    F  H  N  E  T  G  A  N  P  N  P  S  G  V  Q  N  I  L  T  Y 293
TTTTCATAATGAGACTGGCGCCAACCCTAATCCTAGTGGTGTTCAGAATATTCTAACTTA 960

         K
    Q  T  Q  T  A  Q  S  G  Y  Y  N  F  N  F  S  F  L  S  S  F 313
CCAAACACAAACAGCTCAGAGTGGTTATTATAATTTTAATTTTTCCTTTCTGAGTAGTTT 1020


    V  Y  K  E  S  N  F  M  Y  G  S  Y  H  P  S  C  N  F  R  L 333
TGTTTATAAGGAGTCTAATTTTATGTATGGATCTTATCACCCAAGTTGTAATTTTAGACT 1080


    E  T  I  N  N  G  L  W  F  N  S  L  S  V  S  I  A  Y  G  P 353
AGAAACTATTAATAATGGCTTGTGGTTTAATTCACTTTCAGTTTCAATTGCTTACGGTCC 1140

                                  K
    L  Q  G  G  C  K  Q  S  V  F  S  G  R  A  T  C  C  Y  A  Y 373
TCTTCAAGGTGGTTGCAAGCAATCTGTCTTTAGTGGTAGAGCAACTTGTTGTTATGCTTA 1200


    S  Y  G  G  P  S  L  C  K  G  V  Y  S  G  E  L  D  L  N  F 393
TTCATATGGAGGTCCTTCGCTGTGTAAAGGTGTTTATTCAGGTGAGTTAGATCTTAATTT 1260


    E  C  G  L  L  V  Y  V  T  K  S  G  G  S  R  I  Q  T  A  T 413
TGAATGTGGACTGTTAGTTTATGTTACTAAGAGCGGTGGCTCTCGTATACAAACAGCCAC 1320

                  Q  N
    E  F  F  V  I  T  R  H  N  Y  N  N  I  T  L  N  T  C  V  D 433
TGAACCGCCAGTTATAACTCGACACAATTATAATAATATTACTTTAAATACTTGTGTTGA 1380


    Y  N  I  Y  G  R  T  G  Q  G  F  I  T  N  V  T  D  S  A  V 453
TTATAATATATATGGCAGAACTGGCCAAGGTTTTATTACTAATGTAACCGACTCAGCTGT 1440


    S  Y  N  Y  L  A  D  A  G  L  A  I  L  D  T  S  G  S  I  D 473
TAGTTATAATTATCTAGCAGACGCAGGTTTGGCTATTTTAGATACATCTGGTTCCATAGA 1500

                           N
    I  F  V  V  Q  G  E  Y  G  L  T  Y  Y  F  V  N  F  C  E  D 493
CATCTTTGTTGTACAAGGTGAATATGGTCTTACTTATTATAAGGTTAACCCTTGCGAAGA 1560


    V  N  Q  Q  F  V  V  S  G  G  F  L  V  G  I  L  T  S  R  N 513
TGTCAACCAGCAGTTTGTAGTTTCTGGTGGTAAATTAGTAGGTATTCTTACTTCACGTAA 1620
```

```
      E   T   G   S   Q   L   L   E   N   Q   F   Y   I   K   I   T   N   G   T   R 533
    TGAGACTGGTTCTCAGCTTCTTGAGAACCAGTTTTACATTAAAATCACTAATGGAACACG 1680


      R   F   R   R . S   I   T   E   N   V   A   N   C   P   Y   V   S   Y   G   K 553
    TCGTTTTAGACGTTCTATTACTGAAAATGTTGCAAATTGCCCTTATGTTAGTTATGGTAA 1740


      F   C   I   K   P   D   G   S   I   A   T   I   V   P   K   Q   L   E   Q   F 573
    GTTTTGTATAAAACCTGATGGTTCAATTGCCACAATAGTACCAAAACAATTGGAACAGTT 1800

                  F
      V   A   P   L   L   N   V   T   E   N   V   L   I   P   N   S   F   N   L   T 593
    TGTGGCACCTTTACTTAATGTTACTGAAAATGTGCTCATACCTAACAGTTTTAATTTAAC 1860


      V   T   D   E   Y   I   Q   T   R   M   D   K   V   Q   I   N   C   L   Q   Y 613
    TGTTACAGATGAGTACATACAAACGCGTATGGATAAGGTCCAAATTAATTGTCTGCAGTA 1920

              S                           K
      V   C   G   N   S   L   D   C   R   D   L   F   Q   Q   Y   G   P   V   C   D 633
    TGTTTGTGGCAATTCTCTGGATTGTAGAGATTTGTTTCAACAATATGGGCCTGTTTGTGA 1980

                              V
      N   I   L   S   V   V   N   S   I   G   Q   K   E   D   M   E   L   L   N   F 653
    CAACATATTGTCTGTAGTAAATAGTATTGGTCAAAAAGAAGATATGGAACTTTTGAATTT 2040

                                          V
      Y   S   S   T   K   P   A   G   F   N   T   P   F   L   S   N   V   S   T   G 673
    CTATTCTTCTACTAAACCGGCTGGTTTTAATACACCATTTCTTAGTAATGTTAGCACTGG 2100

                          N           R   K           L
      E   F   N   I   S   L   L   L   T   T   P   S   S   P   R   R   R   S   F   I 693
    TGAGTTTAATATTTCTCTTCTGTTAACAACTCCTAGTAGTCCTAGAAGGCGTTCTTTTAT 2160

                                                  N
      E   D   L   L   F   T   S   V   E   S   V   G   L   P   T   D   D   A   Y   K 713
    TGAAGACCTTCTATTTACAAGCGTTGAATCTGTTGGATTACCAACAGATGACGCATACAA 2220

                          F
      N   C   T   A   G   P   L   G   F   L   K   D   L   A   C   A   R   E   Y   N 733
    AAATTGCACTGCAGGACCTTTAGGTTTTCTTAAGGACCTTGCGTGTGCTCGTGAATATAA 2280

                                                      A
      G   L   L   V   L   P   P   I   I   T   A   E   M   Q   T   L   Y   T   S   S 753
    TGGTTTGCTTGTGTTGCCTCCCATTATAACAGCAGAAATGCAAACTTTGTATACTAGTTC 2340


      L   V   A   S   M   A   F   G   G   I   T   A   A   G   A   I   P   F   A   T 773
    TCTAGTAGCTTCTATGGCTTTTGGTGGTATTACTGCAGCTGGTGCTATACCTTTTGCCAC 2400


      Q   L   Q   A   R   I   N   H   L   G   I   T   Q   S   L   L   L   K   N   Q 793
    ACAACTGCAGGCTAGAATTAATCACTTGGGTATTACCCAGTCACTTTTGTTGAAGAATCA 2460
```

```
            H
  E  K  I  A  A  S  F  N  K  A  I  G  R  M  Q  E  G  F  R  S 813
AGAAAAAATTGCTGCTTCCTTTAATAAGGCCATTGGTCGTATGCAGGAAGGTTTTAGAAG 2520


                                    S
  T  S  L  A  L  Q  Q  I  Q  D  V  V  N  K  Q  S  A  I  L  T 833
TACATCTCTAGCATTACAACAAATTCAAGATGTTGTTAATAAGCAGAGTGCTATTCTTAC 2580


  E  T  M  A  S  L  N  K  N  F  G  A  I  S  S  V  I  Q  E  I 853
TGAGACTATGGCATCACTTAATAAAAATTTTGGTGCTATTTCTTCTGTGATTCAAGAAAT 2640


          F
  Y  Q  Q  L  D  A  I  Q  A  N  A  Q  V  D  R  L  I  T  G  R 873
CTACCAGCAACTTGACGCCATACAAGCAAATGCTCAAGTGGATCGTCTTATAACTGGTAG 2700


                                    Y
  L  S  S  L  S  V  L  A  S  A  K  Q  A  E  H  I  R  V  S  Q 893
ATTGTCATCACTTTCTGTTTTAGCATCTGCTAAGCAGGCGGAGCATATTAGAGTGTCACA 2760


  Q  R  E  L  A  T  Q  K  I  N  E  C  V  K  S  Q  S  I  R  Y 913
ACAGCGTGAGTTAGCTACTCAGAAAATTAATGAGTGTGTTAAGTCACAGTCTATTAGGTA 2820


  S  F  C  G  N  G  R  H  V  L  T  I  P  Q  N  A  P  N  G  I 933
CTCCTTTTGTGGTAATGGACGACATGTTCTAACCATACCGCAAAATGCACCTAATGGTAT 2880


  V  F  I  H  F  S  Y  T  P  D  S  F  V  N  V  T  A  I  V  G 953
AGTGTTTATACACTTTTCTTATACTCCAGATAGTTTTGTTAATGTTACTGCAATAGTGGG 2940


  F  C  V  K  P  A  N  A  S  Q  Y  A  I  V  F  A  N  G  R  G 973
TTTTTGTGTAAAGCCAGCTAATGCTAGTCAGTATGCAATAGTACCCGCTAATGGTAGGGG 3000


  I  F  I  Q  V  N  G  S  Y  Y  I  T  A  R  D  M  Y  M  P  R 993
TATTTTTATACAAGTTAATGGTAGTTACTACATCACAGCACGAGATATGTATATGCCAAG 3060


          V
  A  I  T  A  G  D  I  V  T  L  T  S  C  Q  A  N  Y  V  S  V 1013
AGCTATTACTGCAGGAGATATAGTTACGCTTACTTCTTGTCAAGCAAATTATGTAAGTGT 3120


  N  K  T  V  I  T  T  F  V  D  N  D  D  F  D  F  N  D  E  L 1033
AAATAAGACCGTCATTACTACATTCGTAGACAATGATGATTTTGATTTTAATGACGAATT 3180


  S  K  W  W  N  D  T  K  H  E  L  P  D  F  D  K  F  N  Y  T 1053
GTCAAAATGGTGGAATGACACTAAGCATGAGCTACCAGACTTTGACAAATTCAATTACAC 3240


  V  F  I  L  D  I  D  S  E  I  D  R  I  Q  G  V  I  Q  G  L 1073
AGTACCTATACTTGACATTGATAGTGAAATTGATCGTATTCAAGGCGTTATACAGGGTCT 3300
```

FIGURE 1 (cont.)

**0221609**

```
  N  D  S  L  I  D  L  E  K  L  S  I  L  K  T  Y  I  K  W  P 1093
TAATGACTCTTTAATAGACCTTGAAAAACTTTCAATACTCAAAACTTATATTAAGTGGCC 3360


  W  Y  V  W  L  A  I  A  F  A  T  I  I  F  I  L  I  L  G  W 1113
TTGGTATGTGTGGTTAGCCATAGCTTTTGCCACTATTATCTTCATCTTAATACTAGGATG 3420


  V  F  F  M  T  G  C  C  G  C  C  C  G  C  F  G  I  M  P  L 1133
GGTTTTCTTCATGACTGGATGTTGTGGTTGTTGTTGTGGATGCTTTGGCATTATGCCTCT 3480


  M  S  K  C  G  K  K  S  S  Y  Y  T  T  F  D  N  D  V  V  T 1153
AATGAGTAAGTGTGGTAAGAAATCTTCTTATTACACGACTTTTGATAACGATGTGGTAAC 3540


             Y
  E  Q  N  R  P  K  K  S  V  *  *                            1162
TGAACAAAACAGACCTAAAAAGTCTGTTTAATGATTCAAAGTCCCACGTCCTTCCTAATA 3600
                        M  I  Q  S  P  T  S  F  L  I  RNAD
```

DUPHAR INTERNATIONAL RESEARCH B.V.

DIR 0370

## FIGURE 2

0221609

```
D1466  MWAQLLSVVTLLFAL*SECSIVGENYTYYYQSQFRPPNGWHKHGGAYLVTNET
D274   ----------------------------------------------------
D207   MLERSLLLATLLSALCSANLFGNNSYVYYYQSAFRPPDGWHLHGGAEDVVNQS
H120   MLVTPLLLVTLLCALCSAALYDSSSYVYYYQSAFRPPDGWHLHGGAYAVVNIS
M42    MLVTPLLLVTLLCALCSAVLYDSSSYVYYYQSAFRPPSGWHLQGGAYAVVNIS
M41    MLVTPLLLVTLLCVLCSAALYDSSSYVYYYQSAFRPPNGWHLHGGAYAVVNIS


DISYN*GVS**CTVGTIKGGIVINESAIS*FVTKTPIAWSANGVCTTYCNYSSLYVFVTH
-----------------------------------------------------------
TYSSNAGTT*GCTAGAIYWSKNFSAASVAMTAPQNGMSWSTEQFCTAHCNFTDFVVFVTH
SESNNAGSSIGCTVGIIHGGRVVNASSIAMTAPSSGMAWSSSQFCTAYCNFSDTTVFVTH
SEFNNAGSSSGCTVGIIHGGRVVNASSIAMTAPSSGMAWSSSQFCTAHCNFSDTTVFVTH
SESNNAGSSPGCIVGTIHGGRVVNASSIAMTAPSSGMAWSSSQFCTAHCNFSDTTVFVTH


CGGSGHTSC*IINTNRIGEIVLG*VKDFSGNWIYNRTIKAIG*PYSKFTAWQCLANFTSVFLNGNL
----------------------------------------------------------------
CYKNGPGSCPLTGLIPQYHIRISAMKNSSLF**YNLTVAVTKYPRFK*SL*QCVNHMTSVYLNGDL
CYKHV*G*CPITGMLQQHSIRVSAMKNGQLF**YNLTVSVAKYPTFK*SF*QCVNNLTSVYLNGDL
CYKHG*G*CPLTGMLQQNLIRVSAMKNGQLF**YNLTVSVAKYPTFR*SF*QCVNNLTSVYLNGDL
CYKYD*G*CPITGMLQKNFLRVSAMKNGQLF**YNLTVSVAKYPTFK*SF*QCVNNLTSVYLNGDI


VYSSNFTEDVAAGGVYAKSVNGLKRRIMKDTDVLAYFVNGTAVEVIVCDDSPRGRLACQY
-----------------------------------------------------------
VFTSNETKDVSAAGVYAKAGNPITRKVMREVRVLAYFVNGTAVDVILCDGSPRGLLACQY
VYTSNETTDVTSAGVYFKAGGPITYKVMREVRALAYFVNGTAQDVILCDGSPRGLLACQY
VYTSNETIDVTSAGVYFKAGGPITYKVMREVKALAYFVNGTAQDVILCDGSPRGLLACQY
VYTSNETTDVTSAGVYFKAGGPITYKVMRKVKALAYFVNGTAQDVILCDGSPRGLLACQY


NTGNFTDGLYPFVSYNVVNESVVVYEVISTRTYGKLNNITFHNETGAPPAGSNVANFIKY
---------------------------------------------------AGSNVANFIKY
NTGNFSDGFYPFTNSSLVKEKFIVYRESSVNTTLELTNFTFSNVSNATPNTGGVQTIQLY
NTGNFTDGFYPFTNSSLVKQKFIVYRENSVNTTETLHNFTFHNETGANPNPSGVQNIQTY
NTGNFSDGFYPFTNSSLVKQKFIVYRENSVNTTCTLHNFIFHNETGANPNPSGVQNIQTY
NTGNFSDGFYPFINSSLVKQKFIVYRENSVNTTFTLHNFTFHNETGANPNPSGVQNILTY


QTHVVPEGFVRLNFSFLSTYRYQESDFTYGSYHKACNFRLESINNGLMFNTLSVSISYGP
QTHVVPEGFVRLNFSFLSTYRYQESDFTYGSYHKACNFRLESINNGLMFNTLSVSISYGP
QTSTAQSGYYNLNFSFLSSFIYKASDYMYGSYHPSCKFKLETINNGLWFNSLSVSLGYGP
QTQTAQSGYYNFNFSFLSSFVYKESNFMYGSYHPSCNFRLETINNGLWFNSLSVSIAYGP
QTKTAQSGYYNFNFSFLSSFVYKESNFMYGSYHPSCKFRLETINNGLWFNSLSVSIAYGP
QTQTAQSGYYNFNFSFLSSFVYKESNFMYGSYHPSCNFRLETINNGLWFNSLSVSIAYGP


LKGSCKQSVFNRKATCCYAYKYPTNGVQECKGVYNGERNTKFECGLLVFIDKTDGSRIITAE
LKGSCKQSVFNRKATCCYAYKYPTNGVQECKGVYNGERNTKFECGLLVFIDKTDGSRIITAE
IQGGCKQSVFANRATCCYVYSY**NGPSFCKGVYRGELTKSFECGLLVFVTKTDGSRIQTRN
LQGGCKQSVFSGRATCCYAYSY**GGPLLCKGVYSGELDHNFECGLLVYVTKSGGSRIQTAT
LQGGCKQSVFKGRATCCYAYSY**GGPSLCKGVYSGELDHNFECGLLVYVTKSGGSRIQTAT
LQGGCKQSVFSGRATCCYAYSY**GGPSLCKGVYSGELDLNFECGLLVYVTKSGGSRIQTAT
```

1

DUPHAR INTERNATIONAL RESEARCH B.V.

EP 0370

FIGURE 2 (cont.)

**0221609**

```
KPPVYTTNFTNNIVVGKCVNYNIYGRYGQGVISNITTKAFGF**LQGDGLVILDTAGSID
KPPVYTTNFTNNIVVGKCVNYNIYGRYGQGVISNITTKAFGF**LQGDGLVILDTAGSID
EPFTLTQHNYNNITLDRCVEYNIYGRVGQGFITNVTNYAINYNYLADGGMAILDTSGAID
EPPVITQHNYNNITLNTCVDYNIYGRTGQGFITNVTDSAVSYNYLADAGLAILDTSGSID
EPPVITQNNYNNITLNTCVDYNIYGRTGQGFITNVTDSAVSYNYLADAGLAILDTSGSID
EPPVITRHNYNNITLNTCVDYNIYGRTGQGFITNVTDSAVSYNYLADAGLAILDTSGSID


IFSVKDGPLTHYYKINPCNDVNQQYVVSGGNIVGLLTSSNETGSIQLEDQFYIKLTNSTR
IFSVKDGPLTHYYKINPCNDVNQQYVVSGGNIVGLLTSSNETGSIQLEDQFYIKLTNSTR
IFVVQGEYGLNYYKVNPCEDVNQQFVVSGGKLVGILTSRNETGSQPLENQFYIKIINGTR
IFVVQSEYGLNYYKVNPCEDVNQQFVVSGGKLVGILTSRNETGSQLLENQFYIKITNGTR
IFVVQGEYGLNYYKVNPCEDVNQQFVVSGGKLVGILTSRNETGSQLLENQFYIKITNGTR
IFVVQGEYGLTYYKVNPCEDVNQQFVVSGGKLVGILTSRNETGSQLLENQFYIKITNGTR


RHRR
RHRP
RSRR
RFRR
RFRR
RFRR
```

2

FIGURE 3

The Infectious Bronchitis Virus E₂ gene.

■ = highly vari-
able sequence

## DOCUMENTS CONSIDERED TO BE RELEVANT

| Category | Citation of document with indication, where appropriate, of relevant passages | Relevant to claim | CLASSIFICATION OF THE APPLICATION (Int Cl 4) |
|---|---|---|---|
| X | JOURNAL OF GENERAL VIROLOGY, vol. 66, no. 10, October 1985, pages 2253-2258, SGM; M.E.G.BOURSNELL: "Sequencing of coronavirus IBV genomic RNA: three open reading frames in the 5'"Unique" region of mRNAD" * pages 2255,2256, first paragraph * | 1-12 | C 12 N 15/00<br>A 61 K 39/215<br>C 07 K 13/00<br>C 12 N 1/20<br>C 12 P 21/02 |
| X | JOURNAL OF GENERAL VIROLOGY, vol. 66, no. 4, April 1985, pages 719-726, SGM; M.M.BINNS et al.: "Cloning and sequencing of the gene encoding the spike protein of the coronavirus IBV" * Whole document * | 1-12 | |
| X,P | WO-A-8 605 806 (NATIONAL RESEARCH DEVELOPMENT CORPORATION) * Whole document * | 1-12 | **TECHNICAL FIELDS SEARCHED (Int Cl 4)**<br><br>C 12 N<br>C 12 P<br>A 61 K |
| X | EP-A-0 073 547 (GIST BROCADES N.V.) * Page 2, lines 10-16 * | 2,4-7 | |

--- -/-

The present search report has been drawn up for all claims

| Place of search | Date of completion of the search | Examiner |
|---|---|---|
| THE HAGUE | 26-01-1987 | CUPIDO M. |

# EUROPEAN SEARCH REPORT

**European Patent Office**

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | MODERN APPROACHES TO VACCINES, COLD SPRING HARBOR LABORATORY, 1984, pages 215-218, New York, US; D.CAVANAGH et al.: "Genetically engineered vaccine against avian infectious bronchitis virus with the advantages of current live and killed vaccines" * Page 216 * | 2,4-7 | |

-----

TECHNICAL FIELDS SEARCHED (Int Cl.4)

The present search report has been drawn up for all claims

| Place of search | Date of completion of the search | Examiner |
|---|---|---|
| THE HAGUE | 26-01-1987 | CUPIDO M. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO Form 1503 03 82